(19) Europäisches Patentamt
European Patent Office
Office européen des brevets

(11) Publication number: 0 468 643 A1

(12) EUROPEAN PATENT APPLICATION

(21) Application number: 91305784.0

(51) Int. Cl.[5]: C07C 271/16, C07C 269/06

(22) Date of filing: 26.06.91

(30) Priority: 21.07.90 GB 9016062

(43) Date of publication of application:
29.01.92 Bulletin 92/05

(84) Designated Contracting States:
BE DE ES FR GB IT NL

(71) Applicant: THE BRITISH PETROLEUM COMPANY P.L.C.
Britannic House, 1 Finsbury Circus
London EC2M 7BA(GB)

(72) Inventor: Ambler, Philip William, The B.P. Company p.l.c.
Chertsey Road, Sunbury-on-Thames
Midlesex, TW16 7LN(GB)
Inventor: Stewart, Nevin John, The B.P. Company p.l.c.
Chertsey Road, Sunbury-on-Thames
Midlesex, TW16 7LN(GB)
Inventor: Hodgson, Philip Kenneth Gordon, The B.P. Co. p.l.c
Chertsey Road, Sunbury-on-Thames
Midlesex, TW16 7LN(GB)

(74) Representative: Ryan, Edward Terrence et al
BP INTERNATIONAL LIMITED, Patents Division Chertsey Road
Sunbury-on-Thames Middlesex, TW16 7LN(GB)

# (54) Process for the preparation of acrylates.

(57) (Meth)acrylate esters containing an amine group blocked by a carbonate derivative having, e.g. t-butyl group, are made by reaction of a methyl or ethyl (meth)acrylate with a hydroxy carbamate derivative containing, e.g. a t-butyl group. The hydroxy carbamate derivative may be made by reacting an alkanolamine with an alkyl aryl carbonate in which the alkyl group is, e.g. t-butyl.

EP 0 468 643 A1

This invention relates to a process for the preparation of acrylates, more particularly to protected amino derivatives of acrylates.

Acrylates and methacrylates (hereinafter referred to jointly as (meth)acrylates, containing amine groups are potentially useful reactants for various purposes. However the presence of free reactive amine groups can cause problems during the processing of such compounds, for example by interfering with polymerisation. It has been disclosed that amine groups in (meth)acrylates may be protected or blocked by reaction with suitable protective groups for example the t-butyl groups. These protective groups can be removed subsequently to give a compound with a free amino group.

Finkenaur has described (Polymer Preprints, Am. Chem. Soc., Div. Polym. Chem, 1982, 23(2) pp. 91-92, and PhD dissertion, University of Massachusetts, 1984, abstracted Dissertation Abstracts International 45(1), July 1984) the preparation of N-t-butoxycarbonyl-2-aminoethyl methacrylate by reaction of the hydrochloride salt of 2-aminoethyl methacrylate with 2-(t-butoxycarbonyloxyimino)-2-phenylacetonitrile in the presence of triethylamine. Such a process is unattractive on a larger scale, and there is a need for an alternative process.

Australian patent specification AU-A-79022187 discloses a process for the production of coatings by radiation cross-linking, using a reactive diluent which may be the acrylate of isopropyl beta-hydroxy ethyl carbonate. This may also be named N-isopropoxy carbonyl-2-amino ethyl acrylate. It is made by the esterification of acrylic acid with reaction of N-isopropoxy carbonyl-2-amino ethanol. The amino ethanol derivative was prepared by the reaction of isoproxy carbonyl chloride with amino ethanol.

The isopropoxy carbonyl group is not suitable as a protecting group for amine groups because it cannot be removed easily. The preparation of the N-isopropoxy carbonyl-2-amino ethanol intermediate compound involves the preparation of an organo chlorine compound and it is desirable to avoid routes which make use of organo chlorine compounds. t-butyl chloroform is an unstable compound which needs to be kept below -30° C..

Ragnarsson et al, Acta Chem. Scand. 26 (1972) disclose the base-catalysed reaction of t-butyl phenyl carbonate with amino acids to protect the amine groups of the amino acids. There is no mention of the treatment of amino ethanol.

US 1 927 858 discloses the reaction of ethanolamine with diethyl carbonate. In this reaction a dialkyl carbonate is used and there is no possibility of forming anything other than the ethyl derivative. We have found that when using the corresponding di(t-butoxy)carbonate poor results are obtained. However if a mixed carbonate is used there is the possibility that undesired derivatives will be produced reducing the yield.

We have now found an improved process for making blocked or protected amine (meth)acrylates such as N-t-butoxy carbonyl-2-amino ethyl methacrylate by starting with a carbonate derivative.

We have also found an improved process for making the carbonate derivative from an alkyl aryl carbonate.

According to one aspect of the present invention there is a process for the preparation of a compound of the general formula:

$$CH_2 = CR\text{-}CO_2\text{-}(CH_2)n\text{-}NH\text{-}CO_2\text{-}CR^1R^2R^3 \quad (I)$$

in which R represents a hydrogen atom or a methyl group;
$R^1$ represents a C(1-4) alkyl group having an alpha hydrogen atom; either each of $R^2$ and $R^3$ independently represents a C(1-4) alkyl or alkenyl group, a C(3-8) cycloalkyl group or a phenyl group, or $R^2$ and $R^3$ together with the interjacent carbon atom represent a C(3-8) cycloalkyl group; and
n is an integer from 2 to 6; which comprises reacting a compound of the general formula:

$$CH_2 = CR\text{-}CO_2R^4 \quad (II)$$

in which R has the meaning given for the general formula I and $R^4$ represents a methyl or ethyl group, with a compound of the general formula:

$$HO\text{-}(CH_2)_n\text{-}NH\text{-}CO_2\text{-}CR^1R^2R^3 \quad (III)$$

in which $R^1$, $R^2$, $R^3$ and n have the meanings given for the general formula I.

$R^1$, $R^2$ and $R^3$ may carry inert substituents which do not interfere with the reaction. Thus it may be possible to use fluoro-substituted alkyl.

Preferably R represents a methyl group. Preferably each of $R^1$, $R^2$ and $R^3$ represents a methyl group. Preferably $R^4$ represents a methyl group. Preferably n is 2.

The reaction is preferably carried out at a temperature in the range of from 50 to 120° C, especially 90 to 110° C. Any suitable solvent may be used; typical solvents include hydrocarbons, for example octane, chlorohydrocarbons, for example 1,2-dichloroethane, or ethers, for example di-n-propylether. Preferably however the reaction is carried out in the absence of added solvent. The reaction is suitably carried out in the presence of a transesterification catalyst.

Particularly suitable catalysts include metal alkoxides or oxides, for example lithium hydroxide, the calcium oxide/lithium oxide catalyst described

in GB-A-2163149, titanium tetraisopropoxide, or 1,5,7-triazabicyclo[4.4.0]dec-5-ene and related compounds described in US 4559180. The molar ratio of the compound of the general formula II to the compound of formula III may vary widely, but is preferably in the range of from 1:1 to 5:1.

The compounds of the general formula II are commercially available.

The production of hydroxy alkyl carbonates

According to a further aspect of the invention a new and especially preferred method for the preparation of a compound of the general formula (III), comprises reacting an alkanolamine $HO(CH_2)_nNH_2$ in which n is an integer from 2 to 6, with a compound of the general formula:

$PhO\text{-}CO_2\text{-}CR^1R^2R^3$ (IV)

in which Ph represents a substituted or unsubstituted phenyl group and $R^1$, $R^2$ and $R^3$ have the meanings given for the general formula I.

The phenyl group may be substituted with any inert substituent commonly present in this field of organic synthesis. Especially suitable are electron withdrawing substituents, for example halogen atoms and haloalkyl, cyano and nitro groups. Up to 5 substituents may be present, but preferably the phenyl group is either unsubstituted or has 1 to 3 substituents.

Preferably the alkanolamine is ethanolamine.

The reaction is preferably carried out at a temperature in the range of from 20 to 150 °C, especially from 90 to 110 °C. If desired, the reaction may be carried out in the presence of a suitable solvent, for example a halohydrocarbon such as chloroform. Preferably however the reaction is carried out in the absence of added solvents. The molar ratio of alkanolamine to compound of the general formula (IV) is not critical; it may for example vary from 1:3 to 3:1, especially 1:1.5 to 1.5:1.

The reaction of an alkanolamine with a compound of the general formula IV leads to excellent yields and selectivity. Major advantages of the process are that there is no requirement for the use of a separate solvent, and there is no requirement for the use of a base, both of which might have been expected to be necessary. The high selectivity of the reaction, in which only the PhO group is displaced, is also surprising.

Alternative routes to hydroxyl alkyl carbonates

A second method of preparing a compound of formula III is the reaction of an alkanolamine $HO\text{-}(CH_2)_n\text{-}NH_2$ with a compound of the general formula:

$(R^1R^2R^3c)_2(O.CO.O.CO.O)$ (V)

in which $R^1$, $R^2$ and $R^3$ have the meanings given for the general formula I.

This is a less preferred method because the dialkyl dicarbonate reactant is not readily available on a large scale.

The reaction of the alkanolamine with the compound of the general formula V is preferably carried out at a temperature in the range of from 0 to 75 °C, especially 20 to 60 °C. Any suitable solvent may be used; typical solvents include chlorinated hydrocarbons, for example chloroform or dichloromethane. The molar ratio of alkanolamine to compound of the general formula V may vary widely, but is preferably in the range of from 1:1 to 3:1.

A third method of preparing a compound of formula III is the reaction of an alkanolamine $HO\text{-}(CH_2)_n\text{-}NH_2$ with a compound of formula:

$R^5O\text{-}CO_2\text{-}CR^1R^2R^3$ (VI)

in which $R^5$ represents a $C_1\text{-}C_4$ alkyl group and $R^1$, $R^2$ and $R^3$ have the meanings given for general formula I.

$R^5$ in general formula VI may represent for example be a methyl group. The reaction of alkanolamine with a compound of the general formula VI is preferably carried out in the presence of a strong base. Suitable bases may be inorganic or organic, for example those described above for the reaction of compounds II and III. Alkali metal alkoxides are particularly preferred. The reaction is preferably carried out at a temperature in the range of from 50 to 150 °C, especially 90 to 110 °C. Excess alkanolamine or compound VI may be used as a solvent, or an additional solvent, for example chloroform, may be used.

Compounds of the general formula V and VI are commercially available or can be made by known methods.

The third method for producting compounds of formula III described above has the disadvantage that the yield of the desired compound III tends not to be high.

A consideration of the second and third methods of producing compound III, which is itself an intermediate for producing compound I shows the importance of selecting the appropriate sequence of steps from the many alternatives available in order to arrive at a synthetic route which has any possibility of being commercially viable.

The following Examples illustrate the invention.

Example 1

Preparation of N-t-butoxycarbonylethanolamine

Method 1

t-butyl phenyl carbonate (100 g, 0.515 mol) was added to ethanolamine (33.55 g, 0.55 mol) and the mixture heated at 100 °C for 2 hours. GC analysis indicated essentially complete reaction. Distillation under reduced pressure gave phenol (boiling point 48-50 °C/0.3 mmHg) and the product, N-t-butoxycarbonyl ethanolamine (boiling point 97-99 °C/0.3 mmHg) (69.5 g, 84%).

Method 2

Di-t-butyl-dicarbonate (70 g, 0.32 mol) in chloroform (50 ml) was added dropwise to ethanolamine (19.96 g, 0.33 mol) in chloroform (600 ml), causing a slight temperature rise. After addition the reaction mixture was refluxed (1h), then solvent removal and distillation gave N-t-butoxycarbonylethanolamine as a clear oil (43.8 g, 91%) boiling point 88-91 °C/0.15 mmHg.

Method 3

Ethanolamine (11.1 g, 0.182 mol) and methyl t-butyl carbonate (12 g, 0.091 mol) were heated at 100 °C for three hours under nitrogen in the presence of potassium t-butoxide (102 mg, 0.91 mmol). Gas chromatographic analysis indicated approximately 45% conversion of methyl t-butyl carbonate.

Example 2

Preparation of N-t-butoxycarbonylaminoethyl methacrylate

N-t-butoxycarbonylethanolamine (5 g, 31.1 mmol) and methyl methacrylate (15.53 g, 0.155 mol) were stirred at 20 °C in the presence of 1,5,7-triazabicyclo[4.4.0]dec-5-ene (226 mg, 1.55 mmol). After twenty minutes, gas chromatographic analysis indicated approximately 50% conversion of N-t-butoxycarbonylethanolamine to the product. After this time the reaction rate rapidly decreased giving ultimately a 65% conversion.

Example 3

Preparation of N-t-butoxycarbonylaminoethyl methacrylate

Titanium tetraisopropoxide (270 mg, 0.95 mmol) was added to a mixture of N-t-butoxycarbonylethanolamine (13.08 g, 81.2 mmol), methyl methacrylate (32.48 g, 325 mmol) and 4-methoxyphenol (140 mg, 1.13 mmol). An air sparge was fitted and the mixture refluxed for 3 hours, the pot temperature rising from 105-115 °C over this time. GC indicated 93.5% conversion of starting material.

## Claims

1. A process for the preparation of a compound of the general formula:

$$CH_2=CR\text{-}CO_2\text{-}(CH_2)_n\text{-}NH\text{-}CO_2\text{-}CR^1R^2R^3 \quad (I)$$

in which R represents a hydrogen atom or a methyl group;
$R^1$ represents a C(1-4) alkyl group having an alpha hydrogen atom; either each of $R^2$ and $R^3$ independently represents a C(1-4) alkyl or alkenyl group, a C(3-8) cycloalkyl group or a phenyl group, or $R^2$ and $R^3$ together with the interjacent carbon atom represent a C(3-8) cycloalkyl group; and
n is an integer from 2 to 6; which comprises reacting a compound of the general formula:

$$CH_2=CR\text{-}CO_2R^4 \quad (II)$$

in which R has the meaning given for the general formula I and $R^4$ represents a methyl or ethyl group, with a compound of the general formula:

$$HO\text{-}(CH_2)_n\text{-}NH\text{-}CO_2\text{-}CR^1R^2R^3 \quad (III)$$

in which $R^1$, $R^2$, $R^3$ and n have the meanings given for the general formula I.

2. A process as claimed in claim 1, in which each of $R^1$, $R^2$ and $R^3$ represents a methyl group.

3. A process as claimed in either claim 1 or claim 2, in which the reaction is carried out at a temperature in the range of from 50 to 120 °C.

4. A process as claimed in claim 3, in which the reaction is carried out at a temperature in the range of from 90 to 110 °C.

5. A process as claimed in any one of claims 1 to 4, in which the reaction is carried out in the presence of a basic catalyst.

6. A process for the preparation of a compound of the general formula:

$$HO\text{-}(CH_2)_n\text{-}NH\text{-}CO_2\text{-}CR^1R^2R^3 \quad (III)$$

in which $R^1$ represents a C(1-4) alkyl group having an alpha hydrogen atom, either each of $R^2$ and $R^3$ independently represents a C(1-4) alkyl or alkenyl group, a C(3-8) cycloalkyl

group or a phenyl group, or $R^2$ and $R^3$ together with the interjacent carbon atom represent a C-(3-8) cycloalkyl group, and n is an integer from 2 to 6, which comprises reacting an alkanolamine $HO(CH_2)_nNH_2$ in which n is an integer from 2 to 6, with a compound of the general formula:

$PhO\text{-}CO_2\text{-}CR^1R^2R^3$ (IV)

in which Ph represents a substituted or unsubstituted phenyl group and $R^1$, $R^2$ and $R^3$ have the meanings given for the general formula III.

**7.** A process as claimed in claim 6, in which each of $R^1$, $R^2$ and $R^3$ represents a methyl group.

**8.** A process as claimed in either claim 6 or claim 7, in which the reaction is carried out at a temperature in the range of from 20 to 150 °C.

**9.** A process as claimed in claim 8, in which the reaction is carried out at a temperature in the range of from 90 to 110 °C.

**10.** A process as claimed in any one of claims 1 to 5, in which the compound of the general formula III has been prepared by a process as claimed in any one of claims 6 to 9.

European Patent Office

# EUROPEAN SEARCH REPORT

Application Number

EP 91305784.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE - B - 1 150 973 (KREWEL-LEUFFEN) * Totality * -- | 6,7 | C 07 C 271/16<br>C 07 C 269/06 |
| D,A | US - A - 1 927 858 (H. ULRICH et al.) * Totality * -- | 6,7 | |
| D,A | DIVISION OF POLYMER CHEMISTRY, vol. 23, No. 2, September 1982 A.L. FINKENAUR et al. "Synthesis and characterization of vinyl pentapolymers possessing hemoglobin functions" * Pages 91,92 * -- | 1,2 | |
| D,A | US - A - 4 559 180 (M.J. GREEN) * Examples * ---- | 1,5 | |
| | | | **TECHNICAL FIELDS SEARCHED (Int. Cl.5)** |
| | | | C 07 C 269/00<br>C 07 C 271/00 |

The present search report has been drawn up for all claims

| Place of search | Date of completion of the search | Examiner |
|---|---|---|
| VIENNA | 09-10-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)